# EUROPEAN PATENT APPLICATION

(11) **EP 1 770 394 A2**
(43) Date of publication of application: **04.04.2007**
(21) Application number: 06019436.2
(22) Date of filing: 18.09.2006
(51) Int. Cl.: G01N 31/22

(54) **Composition for measuring residual chlorine concentration**

(30) Priority: 29.09.2005 JP 2005283546
(71) Applicant: MIURA CO., LTD., Matsuyama-shi Ehime-ken (JP)
(72) Inventor: Mitsumoto, Hiroyuki, Matsuyama-shi Ehime-ken (JP)
(74) Representative: Rees, Alexander Ellison

(57) **Abstract**

To attain a composition for measuring residual chlorine concentration capable of preventing crystallization of a coloring reagent in a chemical solution under temperature conditions of 5°C, provided is a composition for measuring residual chlorine concentration in sample water including one or more coloring reagents selected from the group consisting of a dialkyl benzidine compound and a tetraalkyl benzidine compound, an acid, and an alcohol compound. The alcohol compound is selected from the group consisting of a monohydric alcohol, a dihydric alcohol, and a trihydric alcohol, for example, and the composition is prepared to contain the alcohol compound in a content by weight of 20 to 100 times that of the coloring reagent.

## Description

### BACKGROUND OF THE INVENTION

### 1. Field of the Invention

The present invention relates to a composition for measuring residual chlorine concentration in sample water, and more specifically, to a one-solution type composition for measuring residual chlorine concentration in sample water by using a coloring reaction.

### 2. Description of the Related Art

A chlorine agent such as sodium hypochlorite is added to domestic water such as city water or well water, or to pool water. The chlorine agent has effects such as disinfection and sterilization due to an oxidative action, but the effects may be reduced in the presence of a suspension, an organic substance, a metal ion, and the like through a reaction of the chlorine agent and those substances. Further, the effects of the chlorine agent may be lost with time in an open system such as a water storage tank or a pool due to diffusion of the chlorine agent into atmosphere. Thus, residual chlorine concentration in water must be measured regularly, to thereby confirm whether or not a predetermined concentration is maintained.

Meanwhile, in a water treatment system employing various filtration membranes such as a microfiltration membrane, an ultrafiltration membrane, a reverse osmosis membrane, and a nanofiltration membrane, the filtration membranes may be oxidized and degraded in the presence of the chlorine agent in feed water. Degradation of the filtration membrane degrades water quality of treated water. Thus, in general, an activated carbon filtration device or a sodium bisulfite (SBS) addition device is provided in upstream of the filtration membrane, to thereby remove the chlorine agent. In this case, the residual chlorine concentration of the feed water allowed to pass through the activated carbon filtration device or the feed water having sodiumbisul f ite addedmust be measured regularly, to thereby confirm whether or not the chlorine agent is assuredly removed.

Up to now, a measurement method employing a coloring reagent such as o-tolidine or N-diethylphenylene diamine (DPD) is widely used for measurement of the residual chlorine concentration in water. In recent years, there is proposed a measurement method employing a dialkyl benzidine compound or a tetraalkyl benzidine compound, which is safer than DPD, as a coloring reagent. For example, JP 2002-350416 A and JP 09-133671 A each describe use of the dialkyl benzidine compound or the tetraalkyl benzidine compound as a coloring reagent.

Formeasurement of the residual chlorine concentration on site, a portable measurement device or a simple measurement kit is often and generally used. However, the portable measurement device or the simple measurement kit requires skill for operation. Further, a user may feel the operation itself to be complex with frequent measurements, and a demand for an automatic measurement device is increasing. The automatic measurement device is constructed to allow an automatic process of a series of operations including a step of sampling water, a step of adding a coloring reagent, and a step of measuring residual chlorine concentration, and realizes continuous measurement.

### SUNIMARY OF THE INVENTION

The automatic measurement device is generally constructed to store a coloring reagent as a chemical solution in the device and to add a predetermined amount of the chemical solution to sample water in a step of adding a coloring reagent. In the case where the chemical solution is consumed and becomes insufficient, a fresh chemical solution is supplied. The automatic measurement device described above may be provided at various positions in accordance with a water system to be monitored. However, the automatic measurement device is rarely provided at a position under temperature control. Thus, the chemical solution must not change under temperature of 5 to 50°C including severe conditions, for example. However, the coloring reagent used for measuring residual chlorine concentration has a feature in that its solubility reduces in an aqueous solution with temperature decrease. Thus, there is a problem, in the case where an ambient temperature decreases to about 5 to 10°C in the winter or the like, in that the coloring reagent in the chemical solution crystallizes, to thereby cause clogging of a supply path of the chemical solution or the like. Thus, a predetermined amount of the chemical solution cannot be added normally to sample water in the step of adding a coloring reagent. Further, there is a problem in that, even when a predetermined amount of the chemical solution is added to the sample water, measurement lacks reliability because a concentration of the coloring reagent in the chemical solution changes.

The present invention has beenmade in view of the circumstances described above, and an object of the present invention is therefore to realize a composition for measuring residual chlorine concentration capable of preventing crystallization of a coloring reagent in a chemical solution under temperature conditions of 5°C.

In order to solve the above-mentioned problem, according to a first aspect of the present invention, there is provided a composition for measuring residual chlorine concentration in sample water, characterized by including: one or more coloring reagents selected from the group consisting of a dialkyl benzidine compound and a tetraalkyl benzidine compound; an acid; and an alcohol compound.

Further, according to a second aspect of the present invention, there is provided a composition for measuring residual chlorine concentration according to the first aspect of the present invention, characterized in that the alcohol compound is included in a content by weight of 20 to 100 times a content by weight of the coloring reagent.

The present invention can realize a composition for measuring residual chlorine concentration capable of preventing crystallization of a coloring reagent in a chemical solution under temperature conditions of 5°C. That is, even under low temperature conditions in the winter or the like, solubility of a dialkyl benzidine compound or a tetraalkyl benzidine compound serving as a coloring reagent increases and crystallization thereof in the chemical solution is prevented due to an action of an alcohol compound. As a result, a predetermined amount of the chemical solution can stably be added to sample water with an automatic residual chlorine concentration measurement device, for example, and a concentration of the chemical solution can be maintained constant, to thereby assure reliability of measured values.

### DESCRIPTION OF THE INVENTION

Hereinafter, an embodiment of the present invention will be described in detail. A composition for measuring residual chlorine concentration (hereinafter, simply referred to as a "composition") according to this embodiment is a one-solution type chemical solution used for optically measuring residual chlorine concentration in sample water includes one or more coloring reagents selected from the group consisting of a dialkyl benzidine compound and a tetraalkyl benzidine compound, an acid, and an alcohol compound.

The dialkyl benzidine compound or the tetraalkyl benzidine compound is each an oxidative chromogenic coloring reagent which has absorption peaks at wavelengths of about 360 to 380 nm, about 450 to 470 nm, and about 640 to 660 nm in a reaction with residual chlorine in acidic range, and which colors to a hue within a range of yellow to blue green, and is represented by the General formula (I) :

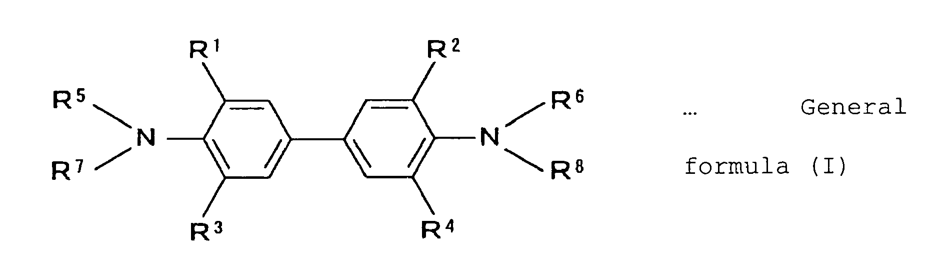

(In the formula: R¹ and R² each independently represent an alkyl group having 1 to 6 carbon atoms; R³ and R⁴ each independently represent an alkyl group having 1 to 6 carbon atoms or a hydrogen atom simultaneously; R⁵ and R⁶ each independently represent an alkyl group having 1 to 8 carbon atoms or a hydrogen atom simultaneously; and R⁷ and R⁸ each independently represent a hydrogen atom, a sulfoalkyl group having 1 to 8 carbon atoms which may have one or more hydroxy groups, or a carboxyalkyl group having 1 to 8 carbon atoms which may have one or more hydroxy groups. R⁷ and R⁸ do not represent hydrogen atoms simultaneously).

Examples of the alkyl group having 1 to 6 carbon atoms as R¹ and R² of the General formula (I) include a methyl group, an ethyl group, a propyl group, an isopropyl group, a butyl group, an isobutyl group, a sec-butyl group, a tert-butyl group, a pentyl group, a 1-methylbutyl group, a 2-methylbutyl group, a 3-methylbutyl group, a 1-ethylpropyl group, a tert-pentyl group, a 1,2-diethylpropyl group, and a hexyl group. Of the alkyl groups, one of R¹ and R² preferably represents a methyl group, and R¹ and R² each more preferably represent a methyl group for the dialkyl benzidine compound. R¹ and R² each preferably represent a methyl group or an ethyl group for the tetraalkyl benzidine compound.

Examples of the alkyl group having 1 to 6 carbon atoms as R³ and R⁴ of the General formula (I) include those described for R¹ and R². Of the alkyl groups, R³ and R⁴ each preferably represent a methyl group or an ethyl group for the tetraalkyl benzidine compound.

Examples of the alkyl group having 1 to 8 carbon atoms as R⁵ and R⁶ of the General formula (I) include those described for R¹ and R², a benzyl group, a phenyl group, and an alkylphenyl group. Of the alkyl groups, R⁵ and R⁶ each preferably represent a methyl group or an ethyl group. In this embodiment, R⁵ and R⁶ each particularly preferably represent a hydrogen atom or an ethyl group.

The sulfoalkyl group having 1 to 8 carbon atoms which may have one or more hydroxy groups as R⁷ and R⁸ of the General formula (I) refers to a derivative in which the same alkyl group as that described for R⁵ and R⁶ is substituted by a sulfonic acid group. A substitution position of the sulfonic acid group is not particularly limited, but the sulfonic acid group is preferably substituted for a terminal of the alkyl group, a benzyl group, or a phenyl group. The sulfoalkyl group may have one or more hydroxy groups, and the substitution positions and number of the hydroxy groups are not particularly limited. In this embodiment, preferred examples of the sulfoalkyl group include a 2-sulfoethyl group, a 3-sulfopropyl group, a 2-hydroxy-3-sulfopropyl group, a 2-hydroxy-2-sulfoethyl group, a 4-sulfobutyl group, and a 2,4-disulfobenzyl group.

The carboxyalkyl group having 1 to 8 carbon atoms which may have one or more hydroxy groups as R⁷ and R⁸ of the General formula (I) refers to a derivative in which the same alkyl group as that described for R⁵ and R⁶ is substituted by a carboxyl group. A substitution position of the carboxyl group is not particularly limited, but the carboxyl group is preferably substituted for a terminal of the alkyl group, a benzyl group, or a phenyl group. The carboxyalkyl group may have one or more hydroxy groups, and the substitution positions and number of the hydroxy groups are not particularly limited. In this embodiment, preferred examples of the carboxyalkyl group include a 2-carboxyethyl group, a 3-carboxypropyl group, a 2-hydroxy-3-carboxypropyl group, a 2-hydroxy-2-carboxyethyl group, a 4-carboxybutyl group, and a 2,4-dicarboxybenzyl group.

Preferred examples of the dialkyl benzidine compound represented by the General formula (I) include:
N,N'-bis(2-sulfoethyl)-3,3'-dimethylbenzidine;
N,N'-bis(3-sulfopropyl)-3,3'-dimethylbenzidine;
N,N'-bis(2-hydroxy-2-sulfoethyl)-3,3'-dimethylbenzidine;
N,N'-bis(2-hydroxy-3-sulfopropyl)-3,3'-dimethylbenzidine;
N,N'-bis(4-sulfobutyl)-3,3'-dimethylbenzidine;
N,N'-bis(3-sulfopropyl)-N,N'-diethyl-3,3'-dimethylbenzidine;
N,N'-bis(2,4-disulfobenzyl)-3,3'-dimethylbenzidine; and alkali metal salts thereof. Of the compounds, a compound in the form of a sodium salt is particularly preferably used because the compound has high water solubility and is hardly crystallized at normal temperatures.

Preferred examples of the tetraalkyl benzidine compound represented by the General formula (I) include:
N-(2-sulfoethyl)-3,3',5,5'-tetramethylbenzidine;
N-(3-sulfopropyl)-3,3',5,5'-tetramethylbenzidine;
N-(4-sulfobutyl)-3,3',5,5'-tetramethylbenzidine;
N-(2-hydroxy-2-sulfoethyl)-3,3',5,5'-tetramethylbenzidine;
N-(2-hydroxy-3-sulfopropyl)-3,3',5,5'-tetramethylbenzidine;
N-(2,4-disulfobenzyl)-3,3',5,5'-tetramethylbenzidine;
N,N'-bis(2-sulfoethyl)-3,3',5,5'-tetramethylbenzidine;
N,N'-bis(3-sulfopropyl)-3,3',5,5'-tetramethylbenzidine;
N,N'-bis(4-sulfobutyl)-3,3',5,5'-tetramethylbenzidine;
N,N'-bis(2-hydroxy-2-sulfoethyl)-3,3',5,5'-tetramethylbenzidine;
N,N'-bis(2-hydroxy-3-sulfopropyl)-3,3',5,5'-tetramethylbenzidine;
N,N'-bis(2,4-disulfobenzyl)-3,3',5,5'-tetramethylbenzidine; and alkali metal salts thereof. Of the compounds, a compound in the form of a sodium salt is particularly preferably used because the compound has high water solubility and is hardly crystallized at normal temperatures.

In general, the dialkyl benzidine compound and the tetraalkyl benzidine compound are each used alone, but two or more kinds thereof may be mixed and used.

A content of the coloring reagent in the composition is not particularly limited and is generally set based on a volume of the sample water, an upper limit of a measurable residual chlorine concentration, and an addition amount of the composition to the sample water. That is, the coloring reagent is mixed in an amount allowing an equivalent reaction of the coloring reagent and residual chlorine when a predetermined amount of the composition is added to the sample water containing a residual chlorine amount determined from a product of the volume of the sample water and the upper limit of measurable residual chlorine concentration. For a case employing N,N'-bis(2-hydroxy-3-sulfopropyl)-3,3'-dimethylbenzidine disodium salt as a coloring reagent, for example, a content of the coloring reagent is set to 0.1 to 0.5 wt% with respect to the volume of the sample water of 4 ml, the upper limit of measurable residual chlorine concentration of 0.5 to 2.5 mg/l (Cl₂ equivalents), and the addition amount of the composition to the sample water of 30 µm.

The acid to be included in the composition is used for adjusting pH of the composition within acidic range. To be specific, the composition has preferably pH 3.5 or less, more preferably pH 3.0 or less, and particularly preferably pH 1.9 or less. The composition is adjusted within acidic range, in particular, to pH 3.5 or less, to thereby suppress sequential increase of absorbance at about a maximum absorption wavelength with time upon coloring of the coloring reagent even in the case where the composition is stored for a long period of time under temperature conditions of 5 to 50°C and allow stable measurement of the residual chlorine concentration. As the acid, an inorganic acid or an organic acid may be used alone, or a combination thereof may arbitrarily be used. Examples of the inorganic acid include acids each exhibiting no oxidative property or reductive property such as sulfuric acid, hydrochloric acid, and phosphoric acid. Examples of the organic acid include citric acid, acetic acid, succinic acid, and oxalic acid.

The alcohol compound to be included in the composition is used for increasing solubility of the coloring reagent at low temperatures and preventing crystallization. As the alcohol compound, a monohydric alcohol or a polyhydric alcohol may be used alone, or a combination thereof may arbitrarily be used. The polyhydric alcohol is preferably a dihydric alcohol or a trihydric alcohol from viewpoints of preventing phase separation in the composition and maintaining a uniformly mixed state with the coloring reagent. The alcohol compound is preferably a lower alcohol compound having 1 to 3 carbon atoms from the same viewpoints. Preferred examples of the alcohol compound in this embodiment include: ethanol or 1-propanol as a monohydric alcohol; ethane-1,2-diol or propane-1,2-diol as a dihydric alcohol; and propane-1,2,3-triol as a trihydric alcohol.

In general, the composition according to this embodiment contains the alcohol compound in a content by weight of preferably 20 to 100 times that of the coloring reagent, and more preferably 30 to 60 times that of the coloring reagent. In the case where the content by weight of the alcohol compound is less than 20 times that of the coloring reagent, a crystallized product easily forms in a short period of time when the temperature of the chemical solution decreases to 5°C, to thereby cause clogging of a supply path of the chemical solution or the like or change the concentration of the coloring reagent in the chemical solution. In contrast, in the case where the content by weight of the alcohol compound is more than 100 times that of the coloring reagent, an excess amount of the alcohol compound does not contribute to preventing formation of the crystallized product and thus is not economical. In the case where the content of the alcohol compound is more than 80 wt% in the composition, uniform mixing of the sample water and the chemical solution requires time and a coloring reaction may be inhibited. Thus, for setting the content of the alcohol compound to 80 wt% or less in the composition, the content of the coloring reagent is desirably set to 0.8 wt% or less in advance.

In this embodiment, an alkali metal salt of an acid may be included as required for finely adjusting the pH of the composition. Examples of the alkali metal salt of an acid include trisodium phosphate, sodium phosphate, and sodium citrate. In the case where the composition is set to pH 1.0 or less, in particular, to pH 0.6, for example, the composition may easily be adjusted to desired pH by preparing a composition having less than pH 0. 6 by using sulfuric acid and phosphoric acid and then adding trisodium phosphate.

In the case where the composition contains phosphoric acid or a phosphate, the phosphoric acid or the phosphate may form a complex with an interfering metal ion such as a reductive metal ion such as Fe²⁺ or an oxidative metal ion such as Cr⁶⁺ or Fe³⁺ in the sample water for masking the interfering metal ion.

The composition may further contain a dye with a predetermined hue as required for optically determining whether or not the composition is normally added to the sample water. The dialkyl benzidine compound and the tetraalkyl benzidine compound are each a compound exhibiting no absorption (i.e., not coloring) when the residual chlorine concentration is 0. Thus, the dye is included in the composition, to thereby confirm an addition state of the composition based on the degree of coloring of the sample water even when the residual chlorine concentration is 0. The kind of dye is not particularly limited as long as the dye exhibits absorption at a wavelength excluding a measurement wavelength. In the case where the measurement wavelength is set to about 640 to 660 nm, for example, a synthetic dye such as New Coccin (CI Acid Red 18) or Acid Red (CI Acid Red 52) may be used.

The content of the dye in the composition is not particularly limited, but may arbitrarily be set within a range of 0.01 to 0.5 wt% from the viewpoints of coloring property, solubility, and economic efficiency, in general.

The composition may further contain a surfactant as required for preventing contamination on an optical measurement cell. As the surfactant, a non-ionic surfactant or an anionic surfactant is preferably used from the viewpoints of having a relatively low critical micelle concentration (CMC) and forming no insoluble suspension by bonding with an organic substance in the sample water. Examples of the non-ionic surfactant that can be used include polyoxyethylene alkyl ether, polyalkylene alkyl ether, polyoxyethylene distyrenated phenyl ether, and a higher alcohol-based non-ionic surfactant. An example of the anionic surfactant that can be used is an alkyldiphenyl ether sulfonic acid salt.

The content of the surfactant in the composition is not particularly limited, but may arbitrarily be set within a range of 0.1 to 5 wt% from the viewpoints of solubility and economic efficiency, in general.

The composition according to this embodiment may be produced by uniformly mixing the coloring reagent, the acid, the alcohol compound, and other additives (such as the dye, the surfactant, and the alkali metal salt of an acid). For example, the composition may be produced by dissolving the coloring reagent and the alcohol compound in an aqueous solution of the acid, and dissolving the alkali metal salt of an acid, the dye, and the surfactant thereinto as required. The composition may be used for measurement of the residual chlorine concentration as a one-solution type chemical solution.

The composition according to the present invention may be used for measurement of the residual chlorine concentration in water. The kind of water containing residual chlorine is not particularly limited, and the composition may widely be used for domestic water, pool water, industrial feed water, industrial process water, and the like.

For measurement of the residual chlorine concentration, measurement conditions such as the concentration of the coloring reagent in the composition, the volume of the sample water, the upper limit of measurable residual chlorine concentration, and the addition amount of the composition to the sample water are generally set in advance, and then, the measurement is performed. First, in a measurement process, the composition is added to the sample water got from a water systembeing monitored such that the coloring reagent is included in an amount equivalent to or more than that of residual chlorine determined from a product of the volume of the sample water and the upper limit of measurable residual chlorine concentration. That is, the composition is added to the sample water such that the coloring reagent in an amount sufficiently covering a measurable range set in advance is included in the sample water. Next, a transmittance (or an absorbance) at about a maximum absorption wavelength (a wavelength which can be used with a commercially available red LED of 665 nm or a wavelength which can be used with a commercially available blue LED of 470 nm) upon coloring of the coloring reagent is detected with respect to the sample water containing the composition added thereto. Then, the residual chlorine concentration in the sample water is determined based on a calibration curve showing a relationship between the residual chlorine concentration and the transmittance (or the absorbance) and determined in advance.

As described above, this embodiment can realize the composition for measuring residual chlorine concentration capable of preventing crystallization of the coloring reagent in the chemical solution under temperature conditions of 5°C. That is, even under low temperature conditions in the winter or the like, solubility of the dialkyl benzidine compound or the tetraalkyl benzidine compound serving as the coloring reagent increases and crystallization thereof in the chemical solution is prevented. As a result, a predetermined amount of the chemical solution can stably be added to the sample water with an automatic residual chlorine concentration measurement device, for example, and the concentration of the chemical solution can be maintained constant, to thereby assure reliability of measured values.

### Examples

### Comparative Examples 1 to 7

A one-solution type chemical solution was prepared by using N,N'-bis(2-hydroxy-3-sulfopropyl)-3,3'-dimethylbenzidine disodium salt (available from Doj indo Laboratories, "SAT-3", trade name) as a coloring reagent and mixing various components in a mixing ratio shown in Table 1. In Table 1, a higher alcohol-based non-ionic surfactant (available from Sanyo Chemical Industries, Ltd., "Naroacty HN-100'', trade name) was used as a surfactant. Then, 100 ml of the chemical solution of each of Comparative Examples was stored in a refrigerator set at a temperature of 5°C, and the presence or absence of a crystallized product after 2 days, 7 days, 15 days, 30 days, 45 days, and 60 days were studied. Table 2 shows the results.

**Table 1**

| Mixing components | Mixing ratio (wt%) | | | | | | |
|---|---|---|---|---|---|---|---|
| | Comparative Example 1 | Comparative Example 2 | Comparative Example 3 | Comparative Example 4 | Comparative Example 5 | Comparative Example 6 | Comparative Example 7 |
| N,N'-bis(2-hydroxy-3-sulfopropyl)-3,3' -dimethylbenzidine disodium salt | 0.5 | 0.5 | 0.5 | 0.5 | 0.5 | 0.5 | 0.5 |
| | | | | | | | |
| Sulfuric acid (10%) | 35.25 | 35.25 | 35.25 | 35.25 | 35.25 | 35.25 | 35.25 |
| | | | | | | | |
| Phosphoric acid | 5 | 5 | 5 | 5 | 5 | 5 | 5 |
| | | | | | | | |
| Trisodium phosphate·12H₂O | 4 | 4 | 4 | 4 | 4 | 4 | 4 |
| | | | | | | | |
| Ethanol | 0 | 7.5 | 0 | 0 | 0 | 0 | 0 |
| | | | | | | | |
| Ethane-1,2-diol | 0 | 0 | 7.5 | 0 | 0 | 0 | 0 |
| | | | | | | | |
| Propane-1,2-diol | 0 | 0 | 0 | 2.5 | 5 | 7.5 | 0 |
| | | | | | | | |
| Propane-1,2,3-triol | 0 | 0 | 0 | 0 | 0 | 0 | 7.5 |
| | | | | | | | |
| New Coccin | 0.105 | 0.105 | 0.105 | 0.105 | 0.105 | 0.105 | 0.105 |
| | | | | | | | |
| Surfactant | 2 | 2 | 2 | 2 | 2 | 2 | 2 |
| | | | | | | | |
| Distilled water | 53.145 | 45.645 | 45.645 | 50.645 | 48.145 | 45.645 | 45.645 |

**Table 2**

| Lapsed days | Presence or absence of crystallized product | | | | | | |
|---|---|---|---|---|---|---|---|
| | Comparative Example 1 | Comparative Example 2 | Comparative Example 3 | Comparative Example 4 | Comparative Example 5 | Comparative Example 6 | Comparative Example 7 |
| 2 days | Absent | Absent | Absent | Present | Absent | Absent | Absent |
| | | | | | | | |
| 7 days | Absent | Absent | Absent | Present | Present | Absent | Absent |
| | | | | | | | |
| 14 days | Present | Present | Present | Present | Present | Present | Present |
| | | | | | | | |
| 30 days | Present | Present | Present | Present | Present | Present | Present |
| | | | | | | | |
| 45 days | Present | Present | Present | Present | Present | Present | Present |
| | | | | | | | |
| 60 days | Present | Present | Present | Present | Present | Present | Present |

### Examples 1 to 7

A one-solution type chemical solution was prepared by using N,N'-bis(2-hydroxy-3-sulfopropyl)-3,3'-dimethylbenzidine disodium salt (available from Dojindo Laboratories, "SAT-3", trade name) as a coloring reagent and mixing various components in a mixing ratio shown in Table 3. In Table 3, a higher alcohol-based non-ionic surfactant (available from Sanyo Chemical Industries, Ltd., "Naroacty HN-100", trade name) was used as a surfactant. Then, 100 ml of the chemical solution of each of Examples was stored in a refrigerator set at a temperature of 5°C, and the presence or absence of a crystallized product after 2 days, 7 days, 15 days, 30 days, 45 days, and 60 days were studied. Table 4 shows the results.

**Table 3**

| Mixing components | Mixing ratio (wt%) | | | | | | |
|---|---|---|---|---|---|---|---|
| | Example 1 | Example 2 | Example 3 | Example 4 | Example 5 | Example 6 | Example 7 |
| N,N'-bis(2-hydroxy-3-sulfopropyl)-3,3' -dimethylbenzidine disodium salt | 0.5 | 0.5 | 0.5 | 0.5 | 0.5 | 0.5 | 0.5 |
| | | | | | | | |
| Sulfuric acid (10%) | 35.25 | 35.25 | 35.25 | 35.25 | 35.25 | 35.25 | 35.25 |
| | | | | | | | |
| Phosphoric acid | 5 | 5 | 5 | 5 | 5 | 5 | 5 |
| | | | | | | | |
| Trisodium phosphate·12H₂O | 4 | 4 | 4 | 4 | 4 | 4 | 4 |
| | | | | | | | |
| Ethanol | 10 | 0 | 0 | 0 | 0 | 0 | 0 |
| | | | | | | | |
| Ethane-1,2-diol | 0 | 10 | 15 | 0 | 0 | 0 | 0 |
| | | | | | | | |
| Propane-1,2-diol | 0 | 0 | 0 | 10 | 15 | 20 | 0 |
| | | | | | | | |
| Propane-1,2,3-triol | 0 | 0 | 0 | 0 | 0 | 0 | 10 |
| | | | | | | | |
| New Coccin | 0.105 | 0.105 | 0.105 | 0.105 | 0.105 | 0.105 | 0.105 |
| | | | | | | | |
| Surfactant | 2 | 2 | 2 | 2 | 2 | 2 | 2 |
| | | | | | | | |
| Distilled water | 43.145 | 43.145 | 38.145 | 43.145 | 38.145 | 33.145 | 43.145 |

**Table 4**

| Lapsed days | Presence or absence of crystallized product | | | | | | |
|---|---|---|---|---|---|---|---|
| | Example 1 | Example 2 | Example 3 | Example 4 | Example 5 | Example 6 | Example 7 |
| 2 days | Absent | Absent | Absent | Absent | Absent | Absent | Absent |
| | | | | | | | |
| 7 days | Absent | Absent | Absent | Absent | Absent | Absent | Absent |
| | | | | | | | |
| 14 days | Absent | Absent | Absent | Absent | Absent | Absent | Absent |
| | | | | | | | |
| 30 days | Absent | Absent | Absent | Absent | Absent | Absent | Absent |
| | | | | | | | |
| 45 days | Absent | Absent | Absent | Absent | Absent | Absent | Absent |
| | | | | | | | |
| 60 days | Absent | Absent | Absent | Absent | Absent | Absent | Absent |

### Evaluation

Table 2 reveals that each chemical solution prepared to contain the alcohol compound in a content by weight of 0 to 15 times that of the coloring reagent formed a crystallized product of the coloring reagent in a short period of time of 2 to 14 days under temperature conditions of 5°C. Meanwhile, Table 4 reveals that each chemical solution prepared to contain the alcohol compound in a content by weight of 20 to 40 times that of the coloring reagent formed no crystallized product of the coloring reagent after 60 days under temperature conditions of 5°C. Thus, the results indicate that in the case where the chemical solution contains the alcohol compound in a content by weight of 20 times or more that of the coloring reagent, crystallization of the coloring reagent can be prevented for a long period of time.

## Claims

1. A composition for measuring residual chlorine concentration in sample water, comprising:
one or more coloring reagents selected from the group consisting of a dialkyl benzidine compound and a tetraalkyl benzidine compound;
an acid; and
an alcohol compound.

2. A composition for measuring residual chlorine concentration according to claim 1, wherein the alcohol compound is included in a content by weight of 20 to 100 times a content by weight of the coloring reagent.
